Europäisches Patentamt

European Patent Office

Office européen des brevets

(11) Veröffentlichungsnummer: **0 427 747 B1**

(12) **EUROPÄISCHE PATENTSCHRIFT**

(45) Veröffentlichungstag der Patentschrift: **26.10.94**

(21) Anmeldenummer: **89908428.9**

(22) Anmeldetag: **01.08.89**

(86) Internationale Anmeldenummer:
**PCT/EP89/00901**

(87) Internationale Veröffentlichungsnummer:
**WO 90/01318 (22.02.90 90/05)**

(51) Int. Cl.⁵: **A61K 31/275**

(54) **SUBSTITUIERTE PHENYLACETONITRILE ZUR VERWENDUNG ALS RESISTENZBRECHER IN DER MALARIATHERAPIE.**

(30) Priorität: **06.08.88 DE 3826796**

(43) Veröffentlichungstag der Anmeldung:
**22.05.91 Patentblatt 91/21**

(45) Bekanntmachung des Hinweises auf die
Patenterteilung:
**26.10.94 Patentblatt 94/43**

(84) Benannte Vertragsstaaten:
**AT BE CH DE FR GB IT LI NL**

(56) Entgegenhaltungen:
**WO-A-88/03802**

**Science, volume 235, 20 February 1987,
(Washington, US), S.K. Martin et al. pages
899-901**

**Proc. Natl. Acad. Sci. USA; volume 84, No.
20, 0ctober 1987, (Washington, US), L.W.
Scheibel et al. pages 7310-7314**

(73) Patentinhaber: **BASF Aktiengesellschaft
Carl-Bosch-Strasse 38
D-67063 Ludwigshafen (DE)**

(72) Erfinder: **KEILHAUER, Gerhard
Industriestrasse 20
D-6701 Dannstadt (DE)**
Erfinder: **KÖNIG, Horst
Pariser Strasse 4
D-6700 Ludwigshafen (DE)**
Erfinder: **SCHLICK, Erich
Reiherstrasse 9
D-6701 Otterstadt (DE)**
Erfinder: **SEITZ, Werner
Bismarckstrasse 22 b
D-6831 Planckstadt (DE)**

Am. J. Trop. Med. Hyg., volume 39, No. 1, 1988, The American Society of Tropical Medicine and Hygiene, (Baltimore, US), G.H. Jocobs et al. pages 15-20

Southeast Asian J. Trop. Med. Pub. Health, Volumn 18, No. 2, June 1987, (Bangkok, TH), J. Satayavivad et al. pages 253-258

Biochemical and Biophysical Research, Communications, volumn 155, No. 1, 30 August 1988, Academic Press, Inc., (New York, US), Z. Ye et al. pages 476-481

**Beschreibung**

Die vorliegende Erfindung betrifft die Verwendung von substituierten Phenylacetonitrilen zur Brechung von Resistenzen gegen Antimalariamittel.

Resistenzbildung gegen Chemotherapie, vor allem auch Mehrfachresistenz, erweist sich immer wieder als ein großes medizinisches Problem. Bewährte Arzneimittel können dadurch völlig nutzlos werden, und oft steht keine befriedigende Alternative zur verfügung. Die Wege der Resistenzbildung sind noch weitgehend ungeklärt.

Resistente Zellen sind in der Lage, in sie eingedrungene Medikamente wieder nach draußen zu "pumpen" oder aber chemisch zu entgiften (Spektrum der Wissenschaft, Oktober 1988, Seite 30/31).

Die Beobachtung, daß Verapamil Resistenz in Krebszellen (JP-US 83 624/1983) und Plasmodien (Proc. Nat. Acad. Sci. USA, 83, 7310 (1987), Science, 235, 899 (1987)) zu brechen vermag, hat daher in der Onkologie rasch zu klinischen Anwendungen geführt. Hierbei erwies sich die bekannte cardiovaskuläre Wirksamkeit des Verapamils als ein die praktische Anwendbarkeit behindernder Nebeneffekt. Die erforderlichen hohen Dosen setzen die Anwendung unter besonderen klinischen Kautelen (Intensivstation) voraus bzw. verhindern sie überhaupt.

Es ist weiter bekannt, daß die starken Calciumantagonisten Verapamil und Nifedipin auch die Resistenz von Malariaerregern gegen Chemotherapeutika herabsetzen (WO 88/03802).

Es wurde nun gefunden, daß sich bestimmte substituierte Phenylacetonitrile vorteilhaft zur Resistenzbrechung verwenden lassen.

Gegenstand der Erfindung ist die Verwendung von (-)-(S)-1,7-Bis-(phenyl)-3-methylaza-7-cyano-8-methyl-nonan-hydrochlorid (= S-Emopamil), (+)-1-(3,4-Dimethoxyphenyl)-3-methylaza-7-cyan-7-(3,4,5-trimethoxyphenyl)-8-methylnonan-hydrochlorid (= (+)-Gallopamil) oder 1,7-Bis-(phenyl)-3-aza-7-cyano-8-methyl-nonan-hydrochlorid (= Noremopamil) zur Herstellung von Arzneimitteln zur Brechung von Resistenzen gegen Malariamittel.

Als Malariamittel, deren Resistenzen gebrochen werden, kommen insbesondere folgende in Betracht: Chloroquin, Hydrochloroquin, Chinin, Pyrimethamin, Mefloquin und Primaquin, sowie Kombinationen dieser Substanzen.

Die oben genannten resistenzbrechenden Substanzen können gewünschtenfalls in Form ihrer Salze mit physiologischen Säuren vorliegen. Als physiologisch verträgliche Säuren kommen vorzugsweise in Betracht: Salzsäure, Schwefelsäure, Phosphorsäure, Essigsäure, Zitronensäure, Malonsäure, Salicylsäure, Maleinsäure, Fumarsäure, Bernsteinsäure, Ascorbinsäure, Äpfelsäure, Methansulfonsäure, Milchsäure, Gluconsäure, Glucuronsäure, Amidosulfonsäure, Benzoesäure, Weinsäure.

Die resistenzbrechenden Verbindungen können zusammen mit oder getrennt von den Antimalariamitteln verabfolgt werden. Sie werden in der Regel oral appliziert, während die Antimalariamittel (= Wirkstoffe) oral oder parenteral (z.B. i.v., i.p.) gegeben werden.

Das Verhältnis von resistenzbrechender Verbindung zu Wirkstoff hängt von der zu behandelnden Krankheit, dem Krankheitszustand des Patienten und dem verwendeten Wirkstoff ab. In der Regel beträgt das Verhältnis etwa 1:1 bis 500:1, vorzugsweise 0,1:1 bis 10:1. Dabei werden die resistenzbrechenden Verbindungen in der Regel in einer Menge von 20 bis 2000 mg pro Patient und Tag bei oraler Gabe und 10 bis 300 mg bei intravenöser bzw. 20 bis 500 mg bei intraperitonealer Gabe pro Patient und Tag angewendet. Die Wirkstoffe werden in der Menge appliziert, die auch für die alleinige Gabe dieser Substanzen vorgesehen ist und die z.B. aus der "Rote Liste 1988" bzw. den darin erwähnten wissenschaftlichen Prospekten entnommen werden kann.

Die resistenzbrechenden Substanzen können in Form von Tabletten, Kapseln oder Dragees zur oralen Applikation oder als Injektionslösung zur parenteralen (i.v., i.p., i.m.) Applikation vorliegen. Lösungen können auch infundiert werden. Die Herstellung der Applikationsformen geschieht in bekannter Weise nach üblichen Methoden.

Die Verwendung der substituierten phenylacetonitrile bringt für die Bekämpfung von Malaria insofern Vorteile, als sie eine bessere cardiale und vasculare Verträglichkeit besitzen.

Herstellungsbeispiele

1. 500 mg (-)-S-1,7-Bis-(phenyl)-3-methylaza-7-cyano-8-methyl-nonan-hydrochlorid und 200 mg Chloroquin wurden in 250 ml physiologischer Kochsalzlösung gelöst, sterilisiert und unter sterilen Bedingungen in eine Infusionsflasche gefüllt.

2. Es wurden Tabletten folgender Rezeptur hergestellt:

| ( + )-1-(3,4-Dimethoxyphenyl)-3-methylaza-7-cyan-7-(3,4,5-trimethoxyphenyl)-8-methyl-nonan-hydrochlorid | 100 mg |
|---|---|
| Chloroquin-dihydrogensulfat | 50 mg |
| Hilfsstoffe | 200 mg |

Die Wirksamkeit der Kombinationstherapie bei Malaria wurde in folgenden Testmodellen bestimmt.

Prüfmodell I

Alle Substanzen wurden zunächst alleine und dann in Kombination mit dem Antimalaria-Mittel an humanen Erythrocyten, die mit resistenten Plasmodien (Pl. falciparum) infiziert waren, getestet. Der Einbau von radioaktiv markiertem Hypoxanthin diente als Maß für das Wachstum der Parasiten. Nach der Microdilutions-Technik (Antimicrob. Agents Chemotherapy 16,710 (1979)), wurde dabei für die erfindungsgemäßen Substanzen allein keine oder nur eine geringe Hemmung des Hypoxanthin-Einbaus gefunden. In Kombination mit einem Malariamittel wurde dagegen eine deutlich synergistische Hemmung beobachtet. Die Auswertung erfolgte über den FIC-Index (eine mathematische Repräsentation eines Isobologramms). Der Wert 1,0 bedeutet Additivität der Wirkungen, ein Wert <0,5 Synergismus. Für R-Verapamil und Chloroquin erhält man den Wert 0,56.

$$\text{FIC-Index} = \frac{[A]}{IC_{50A}} + \frac{[B]}{IC_{50B}} \quad (ng/ml)$$

$IC_{50}$ = Konzentration der Prüfsubstanz, die eine 50 %ige Hemmung der Parasiten bewirkt.

[A] = gemessene $IC_{50}$ der Substanzu A in Gegenwart einer definierten (z.B. 500 bis 125 000 ng/ml) Menge der Substanz B.

[B] = gemessene $IC_{50}$ der Substanz B in Gegenwart einer definierten (z.B. 500 bis 125 000 ng/ml) Menge der Substanz A.

$IC_{50A}$ = $IC_{50}$ der Substanz A alleine gegeben

$IC_{50B}$ = $IC_{50}$ der Substanz B alleine gegeben.

Die folgenden Werte wurden in der Kombination mit Chloroquine bestimmt.

| Testsubstanz | FIC-Index |
|---|---|
| R-Verapamil | 0,56 |
| S-Emopamil | 0,19 |
| ( + )-Gallopamil | 0,53 |
| Noremopamil | 0,19 |

Damit konnte gezeigt werden, daß durch den Zusatz der erfindungsgemäß zu verwendenden Substanzen Chloroquin und andere Antimalariamittel auch gegenüber resistenten Malariaerregern wieder hochwirksam werden.

Prüfmodell II

Zur Bestätigung und weiteren Differenzierung der resistenzbrechenden Wirkung wurden Mäuse nach dem in Ann. Trop. Med. Parasit., 72, 23 (1978) beschriebenen Modell (vgl. auch Exp. Parasitol. 17, 89 (1965), Anm. Trop. Med. Parasit. 69, 155 (1975) mit parasitär befallenden Erythrocyten beimpft und 1 Tag nach der Injektion an vier aufeinanderfolgenden Tagen behandelt. Einen Tag nach der Behandlung wurde die Parasitämie bestimmt.

1. Allgemeine Versuchsbedingungen

Eperythozoon-coccoides-freie, männliche, Schweizer Albino-Mäuse, Gewicht zwischen 18 und 20 g, werden für alle Prüfungen benutzt.

Sie werden in Räumen mit kontrollierter Temperatur (22° + 2°C) in Kunststoffkäfigen zu je 5 Mäusen pro Käfig gehalten.

Testparasit

P. yoelii NS, gewonnen aus P. yoelii N: mäßig resistent gegenüber Chloroquin. Erhaltung durch zyklische Passage durch Anopheles stephensi und unter medikamentöser Belastung in der Maus (60 mg/kg s.c. einmal während der Passage) (für nähere Erläuterung siehe Ann. Trop. Med. Parasit., 72, 23 (1978)).

2. Prüfung der schizontoziden Wirkung im Blut

Männliche Mäuse werden intravenös mit parasität befallenden Erythrozyten ($10^7$) vom P. yoelli NS Stamm beimpft. Danach werden die Tiere an vier aufeinanderfolgenden Tagen beginnend am Tag der Infektion einmal täglich behandelt. Die Verbindungen werden mit ®Tween 80 in sterilem, destilliertem Wasser gelöst oder suspendiert und subkutan verabreicht. In Fällen, in denen sich die Zubereitung einer wäßrigen Lösung als äußerst schwierig erweist, wird die Prüfsubstanz zunächst in Dimethylsulfoxid gelöst. Danach werden wäßrige Verdünnungen zum Gebrauch hergestellt. Die Parasitämie wird am Tag nach der letzten Behandlung bestimmt. Die unterdrückung der Parasiten im Vergleich zur unbehandelten Kontrolle, wird mittels Probitanalyse der log-Dosis-Wirkungskurve berechnet.

Beispiele

Beispiel 1

Mäuse, die mit dem chloroquin-resistenten Plasmodium yoelii ssp infiziert waren, wurden mit Chloroquin über 4 Tage subkutan behandelt, wie unter II.2 beschrieben.

Einen Tag nach der Behandlung wurde die Parasitämie bestimmt und mit der Kontrolle verglichen.

Dabei wurden mit einer Tagesdosis von 10,0 mg/kg Testsubstanz in Kombination mit 0,3 mg/kg Chloroquin folgendes Ergebniss erhalten:

| Substanz | % Parasitämie |
|---|---|
| S-Emopamil | 6,7 |
| (+)-Gallopamil | 8,6 |
| A | 1,9 |
| R-Verapamil | 14,2 |

Diese Daten zeigen, daß die drei zuerst genannten Substanzen die Parasitämie deutlich stärker herabsetzen als R-Verapamil.

In der folgenden Tabelle werden die erfindungsgemäßen Substanzen beispielhaft mit R,S-Verapamil hinsichtlich ihrer therapeutisch relevanten Eigenschaften verglichen.

Der AEI (Activity Enhancement Index) ist der Quotient aus der $ED_{90}$ für Chloroquin alleine und der $ED_{90}$ für Zusatz von 10 mg/kg Testsubstanz. Er ist also ein direktes Maß für die Wirkungssteigerung des Malariamittels gegenüber resistenten Erregern bei gleichzeitiger Gabe der erfindungsgemäßen Substanzen.

Weiter umfaßt die Tabelle drei praxisübliche Testmodelle auf cardiovasculäre Effekte. Am Gefäßstreifen der Ratte (rat aortic strip) (1. Modell) wird die Dosis bestimmt, die zu einer 50 %igen Relaxation führt, sie ist ein Maß für die gefäßerweiternde und damit blutdrucksenkende Wirkung einer Substanz. Das 2. Modell untersucht die blutdrucksenkende Wirkung am Ganztier (Ratte), bei intravenöser Gabe).

Das 3. Modell untersucht ebenfalls am Ganztier die Wirkung der Testsubstanz auf das Herz.

Angegeben ist jeweils, wieviel größer die Dosis der Testsubstanz sein darf als bei racemischem Verapamil, um den gleichen caridovasculären Effekt auszulösen.

Der Selektivitätsindex S der Tabelle ist das Produkt dieses Faktors mit dem AEI und beschreibt somit, um wieviel schwächer als beim racemischen Verapamil die cardiovasculären Effekte der Testsubstanz sind, wenn sie in der Resistenzbrechung von Malariamitteln in equieffizienten Dosen eingesetzt werden.

Im Vergleich zu der in der Blutdrucktherapie eingesetzten Dosis von 240 bis 480 mg Verapamil kann die für die Resistenzbrechung therapeutisch nutzbare Dosis der erfindungsgemäß zu verwendenden Substanzen - ebenso wie die des R-Verapamils - anhand dieser Tabelle als caridovasculär unwirksam eingestuft werden.

Tabelle 7

| Substanz | AEI (10 mg/kg) | | Ratten Aortenstreifen $ED_{50}$ | | Blutdrucksenkung $ED_{50}$ | | EKG (AV-Block) Ratte i.v. ED | |
|---|---|---|---|---|---|---|---|---|
| S,R-Verapamil | 2,56 | = 1 | = 1 | S | = 1 | S | = 1 | S |
| R-Verapamil | 4,15 | 1,6x | 6,3 | 10,2x | 3,5 | 8,9x | 4,6 | 7,4x |
| b) S-Emopamil | 4,5 | 1,8x | 7,7 | 13,5 | 2,7 | 4,9x | 2,15 | 3,9x |
| c) (+)-Gallopamil | 4,4 | 1,7x | 12,6 | 21,6x | 3,4 | 5,8x | 4,6 | 7,8x |
| g) Noremopamil | 4,1 | 1,60x | 2,9 | 4,6x | 4,3 | 6,9x | 4,6 | 6,9x |

## Patentansprüche

1. Verwendung von
   (-)-(S)-1,7-Bis-(phenyl)-3-methylaza-7-cyano-8-methyl-nonan-hydrochlorid,
   (+)-1-(3,4-Dimethoxyphenyl)-3-methylaza-7-cyan-7-(3,4,5-trimethoxyphenyl)-8-methylnonan-hydrochlorid oder
   1,7-Bis-(phenyl)-3-aza-7-cyano-8-methyl-nonan-hydrochlorid
   zur Herstellung von Arzneimitteln zur Brechung von Reistenzen gegen Malariamittel.

## Claims

1. The use of
   (-)-(S)-1,7-bis(phenyl)-3-methylaza-7-cyano-8-methylnonane hydrochloride,
   (+)-1-(3,4-dimethoxyphenyl)-3-methylaza-7-cyan-7-(3,4,5-trimethoxyphenyl)-8-methylnonane hydrochloride or
   1,7-bis(phenyl)-3-aza-7-cyano-8-methylnonane hydrochloride
   for the preparation of drugs for breaking down resistance to antimalarials.

## Revendications

1. Utilisation
   de chlorhydrate de (-)-(S)-1,7-bis-(phényl)-3-méthylaza-7-cyano-8-méthylnonane,
   de chlorhydrate de (+)-1-(3,4-diméhoxyphényl)-3-méthylaza-7-cyano-7-(3,4,5-triméhoxyphényl)-8-méthylnonane au
   de chlorhydrate de 1,7-bis-(phényl)-3-aza-7-cyano-8-méthylnonane
   pour la préparation de médicaments destinés à annuler la résistance à des agents antipaludiques.